# EUROPEAN PATENT SPECIFICATION

(11) **EP 4 366 657 B1**
(45) Date of publication and mention of the grant of the patent: **14.05.2025**
(21) Application number: 21773668.5
(22) Date of filing: 08.07.2021
(51) Int. Cl.: A61F 2/00

(54) **IMPROVED REFLEX ARTIFICIAL SPHINCHTER**
VERBESSERTER KÜNSTLICHER REFLEXSPHINCHTER
SPHINCTER ARTIFICIEL À RÉFLEXE AMÉLIORÉ

(43) Date of publication of application: 15.05.2024
(73) Proprietor: Lüleci, Hüseyin, 34805 Beykoz/Istanbul (TR)
(72) Inventor: LÜLECI, Ahmet Melih, 34805 Beykoz/Istanbul (TR); LÜLECI, Hüseyin, 34805 Beykoz/Istanbul (TR)
(74) Representative: Genç Ilhan, Oznur
(86) International application number: PCT/TR2021/050700
(87) International publication number: WO 2023/282860

(56) References cited:
- WO-A1-2018/156092
- US-A- 4 994 020
- US-A- 5 478 305
- US-B2- 8 801 594

## Description

### TECHNICAL FIELD OF THE INVENTION

The present invention generally relates to an apparatus for treating incontinence and more specifically relates an apparatus for providing an inflatable artificial sphincter for control of excretory body passages. The invention provides a novel solution which effectively occludes excretory body passage of a patient even when a sudden pressure increment occurs in the abdomen of a patient to which an artificial sphincter is implanted.

### BACKGROUND OF THE INVENTION

A biological urinary sphincter prevents urinary flow via mucosal coaptation, compression and pressure transmission. On the other hand, an artificial urinary sphincter mimics the biological urinary sphincter by providing a competent bladder outlet during urinary storage and an open unobstructed outlet to permit voluntary urination. Similarly, an artificial rectal sphincter may be used to treat fecal incontinence caused by neurological or muscular dysfunction of an anal sphincter.

A known treatment for some cases of incontinence is to provide a patient with a mechanism to occlude the affected excretory body passage. These mechanisms are typically surgically implanted within the patient's body and are adapted to be operable by the patient to selectively open and occlude the body passage. Inflatable artificial sphincters are well known devices in the state of the art. Inflatable sphincters typically include an inflatable cuff for surrounding the passage to be occluded. Usually a pump cooperatively associated with a fluid reservoir is utilized to transfer fluid into and out of the cuff. As fluid is transferred into the cuff, the cuff inflates and closes the circumscribed body passage.

Artificial urinary sphincters (AUS) known in the state of the art consist of three major parts, namely the fluid reservoir, the cuff and a pump which is usually designated as the control mechanism of the AUS. The pump can be placed in a man's scrotum. It can also be placed underneath the skin in a woman's lower belly, labia or leg. Two conduit tubes connect all three major parts to each other. Use of an extra element, in particular of a conduit tube, increases the implantation time, complexity of the surgery and most importantly, the infection risk of a patient after implantation within the body.

A known problem with the inflatable artificial sphincters existing in the state of the art is the failure of the cuff in effectively occluding the excretory body passage when a sudden pressure increase occurs in the patient's abdomen. A sudden pressure increase may occur when, for instance, the patient laughs, coughs or is burst into laughter and also by way of certain physical movements such as bending the upper body down or when lifting a weight. In such cases, the normal pressure formed in the inflatable cuff may fail to effectively occlude the excretory body passage and excreted fluid which already accumulated behind the cuff or in the bladder may unintentionally leak outside the patient's body.

WO 2018/156092 A1 discloses an artificial sphincter comprising an inflatable occlusion means for occluding a body passage, a stretchable fluid reservoir and a pump means. The pump means has a first port in fluid communication with the occlusion means and a second port in fluid communication with the fluid reservoir for selectively transferring an isotonic fluid from the occlusion means to said reservoir to deflate said occlusion means. Distinguishingly, the artificial sphincter further comprises a pressure compensation balloon which is attached to the fluid reservoir and which, in use, is to be implanted in the abdomen of a patient. However, the artificial sphincter according to this piece of prior art needs to be improved further. The isotonic fluid loaded in the artificial sphincter may become contaminated in either during the surgical implantation or later on in time during use of the same. The contamination usually results in formation of solid particle content in the isotonic liquid. The solid particle content of contamination may clog the flow retarder or any of the first and second check valves. Clogging in the flow retarder results in that the artificial sphincter becomes unfunctional and shall surgically be removed from the patient. Furthermore, the flow retarder as taught by this piece of prior art is very tiny in size, difficult to manufacture and finally, the overall system needs to be simplified in order to avoid complexity and high manufacturing costs.

### OBJECTS OF THE PRESENT INVENTION

Primary object of the present invention is to provide a new artificial sphincter which eliminates the drawbacks of the existing artificial sphincters.

In particular, an object of the present invention is to provide a new control mechanism for an artificial sphincter which effectively occludes the excretory body passage of a patient even when a sudden pressure increase occurs in the abdomen of the patient.

A further object of the present invention is to provide a new artificial sphincter which is simple and easy to manufacture.

A final object of the present invention is to provide a simplified artificial sphincter in which use of a flow retarder is eliminated in the vicinity of the pressure compensation balloon.

### BRIEF DESCRIPTIONS OF THE DRAWINGS

The figures whose brief explanations are herewith provided are solely intended for providing a better understanding of the present invention and are as such not intended to define the scope of protection or the context in which said scope is interpreted in the absence of the description.
Fig. 1 shows a perspective view of an artificial sphincter according to the present invention,
Fig. 2 shows the cross-sectional view of the artificial sphincter of Fig. 1 in the inflated state of the occlusion means,
Fig. 3 shows the cross-sectional view of the artificial sphincter of Fig. 1 in the deflated state of the occlusion means,
Fig. 4 shows the cross-sectional view of the semipermeable check valve given in Fig. 2,
Fig. 5A shows 2-dimensional cross-sectional view of the pressure sensitive check valve given in Fig. 2,
Fig. 5B shows 3D schematic view of the pressure sensitive check valve given in Fig. 2,
Fig. 6 shows a multiplicity of the perspective views of the cratered ball and its annular seating,
Fig. 7 shows a multiplicity of the perspective views of the ball and its recesses seating,
Fig. 8.1 shows a semi-schematical cross-sectional view of a pump for use with the artificial sphincter according to the present invention; at a state when the pump bulb is squeezed. Broken arrows show the flow direction of the pressurizing fluid unless the arrow is marked with an "x".
Fig. 8.2 shows the pump embodiment given in Fig. 8.1 in a state when the pump bulb is released.
Fig. 8.3 shows the pump embodiment given in Fig. 8.1 during inflation of the occlusion means
Fig. 8.4 shows the close-up view of resilient lips of the semi rigid check valve given in Fig. 1, i.e. when the pump bulb is squeezed.
Fig. 8.5 shows the close-up view of the detail "Q" from the Fig. 8.4 i.e. when the pump bulb is squeezed.
Fig. 8.6 shows 2-D view of the check valve having resilient lips during compression of the bulb of the pump

### DETAILED DESCRIPTION OF THE INVENTION

The list of reference numerals used in the appended drawings is as follows;
- 1: artificial sphincter
- 3: pressure sensitive check valve
- 6: pump bulb
- 7: pump
- 8: second tube
- 9: first tube
- 11: balloon holder
- 13: first port
- 14: second port
- 20: occlusion means
- 23: stretchable fluid reservoir
- 24: pressure compensation balloon
- 28: lumen
- 30: semipermeable check valve
- 32: pervious seat
- 33: ball
- 34: nest
- 35: ball
- 36: annular seating
- 37: crater
- 39: recessed seating
- 40: recess
- 41: gap
- 42: ball
- 43: nest
- 44: spring
- 45: cratered ball
- 431: semi-rigid check valve
- 432: resilient lip
- 433: ball
- 434: valve seat
- 435: opening
- 442: check valve

Objects of the present invention are achieved by the features of Claim 1 in which an artificial sphincter (1) which, in use, contains a pressurizing fluid is disclosed. The artificial sphincter (1) comprises an inflatable occlusion means (20) for occluding a body passage, a stretchable fluid reservoir (23), a pump means (7) having a first port (13) in fluid communication with said occlusion means (20) via a first tube (9) and a second port (14) in fluid communication with said fluid reservoir (23) via a second tube (8) for selectively transferring pressurizing fluid from said occlusion means to said reservoir to deflate said occlusion means so that a blocked body passage may be opened. The artificial sphincter (1) further comprises a balloon holder (11) having a lumen (28) which establishes fluid communication in between said fluid reservoir (23) and said second tube (8) and a pressure compensation balloon (24) which is in fluid communication with the lumen of said balloon holder (11) and which, in use, is to be implanted intraabdominal of a patient. The artificial sphincter (1) further comprises a semipermeable check valve (30) which is located in the lumen (28) of said balloon holder (11), wherein said semipermeable check valve (30) always allows fluid flow coming from the second tube (8) to pass towards said stretchable fluid reservoir (23) and wherein said semipermeable check valve (30) only allows a restricted fluid flow from the stretchable fluid reservoir (23) towards the second tube (8). The artificial sphincter (1) has a pressure sensitive check valve (3) in between the pressure compensation balloon (24) and the lumen of said balloon holder (11), wherein said pressure sensitive check valve (3) always allows fluid flow from the pressure compensation balloon (24) towards the lumen (28) so that the fluid contained in the pressure compensation balloon is freely flowable towards the inflatable occlusion means (20) and, in use, a sudden pressure increase which occurs in the abdomen of a patient is instantly conveyed to the inflatable occlusion means (20). The fluid contained in the pressure compensation balloon is freely flowable towards the inflatable occlusion means (20) and, in use, a sudden pressure increase which may occur in the abdomen of a patient can instantly be conveyed to the inflatable occlusion means. Advantageously, the patient is prevented from urinary leak during coughing or laughing which results in the sudden pressure increase in the abdomen.

The occlusion means (20) of the artificial sphincter (1) according to the present invention is sized and shaped to occlude an anal or urethral canal of a human being.

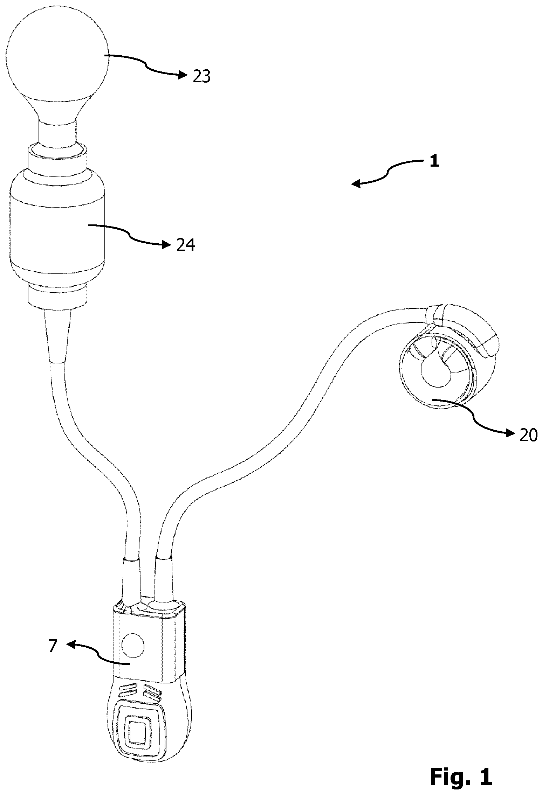

Fig. 1 shows the perspective view of an artificial sphincter (1) according to the present invention. The artificial sphincter (1) has a pump (7) located in between the occlusion means (20) and the stretchable fluid reservoir (23) for selectively transferring the pressurizing fluid. The pump (7) has a compressible bulb (6) for activation by the patient implanted with the artificial sphincter (1). The stretchable fluid reservoir (23) is in fluid communication with a pressure compensation balloon (24).

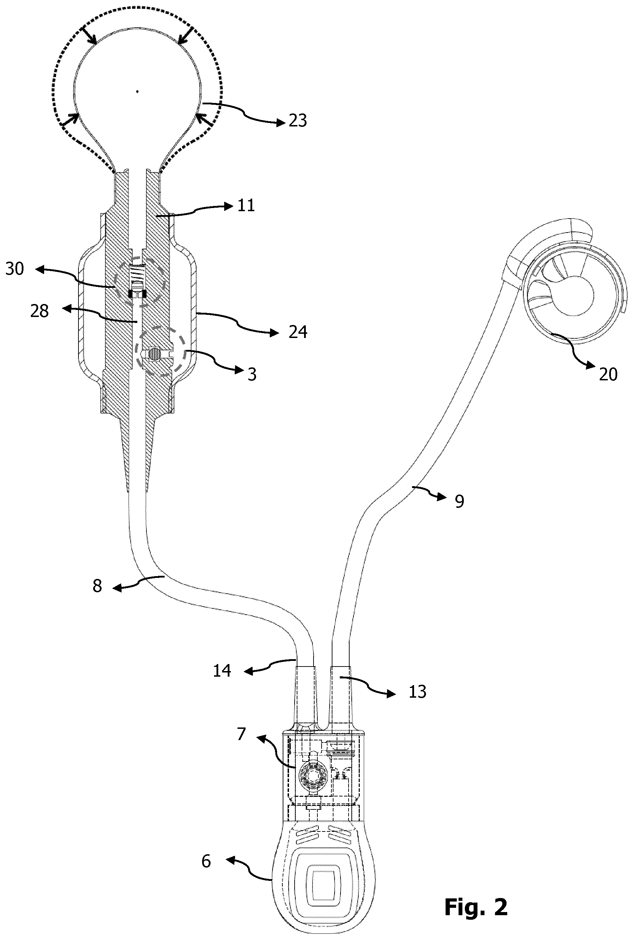

Fig. 2 shows the cross-sectional view of the artificial sphincter of Fig. 1 in the inflated state of the occlusion means (20). The occlusion means (20) is automatically and gradually inflated after it is deflated for allowing urination of the patient. When the bulb is squeezed or otherwise compressed, the pressurizing fluid contained in the bulb is transferred to the stretchable fluid reservoir (23). Once the bulb is squeezed, the ball (433) blocks the pathway towards the occlusion means (20). There is always free flow of the pressurizing fluid in between the pressure compensation balloon (24) and the occlusion means (20). Once the bulb (6) of the pump (7) is squeezed, as shown in Fig. 8a, the pressurizing fluid contained in the bulb (6) is transferred to the stretchable fluid reservoir (23). Once the patient releases the bulb (6), pressurizing fluid flows from the pressure compensation balloon (24) towards the occlusion means (20) and towards the bulb (6). After a plurality of squeezing and releasing, all the fluid contained in the bulb is transferred outside the bulb. In the meantime, the fluid contained in the occlusion means (20) is fully transferred to the stretchable fluid reservoir (23)

In Fig. 2, the arrows shown around the stretchable fluid reservoir (23) implies that the stretchable fluid reservoir (23) shrinks due to transfer of the pressurizing fluid from the stretchable fluid reservoir (23) towards the pressure compensation balloon (24) and hence, towards the occlusion means (20). This transfer of fluid results from the abdominal pressure of the patient. The broken lines around the stretchable fluid reservoir (23) corresponds to state of the stretchable fluid reservoir (23) in the deflated state of the occlusions means (20).

A balloon holder (11) establishes fluid communication in between the stretchable fluid reservoir (23) and the pressure compensation balloon (24). The balloon holder (11) has a lumen (28) at one end of which the stretchable fluid reservoir (23) is attached. The other end of the lumen (28) of the balloon holder (11) is connected to pump (7) via a second tube (8).

The stretchable fluid reservoir (23) is made of a resilient material which may expand by way of stretching such that, in the stretched state, the pressurizing fluid contained in said reservoir (23) has a pressure more than the abdominal pressure of the patient due to stretching of the resilient material. The automatic and gradual transfer of the pressurizing fluid from the pressure compensation balloon (24) towards the occlusions means is activated by the internal pressure of the stretchable fluid reservoir (23).

A semipermeable check valve (30) is located in the lumen (28) of the balloon holder (11). The semipermeable check valve (30) fully allows fluid flow coming from the second tube (8) pass towards the stretchable fluid reservoir (23). However, the semipermeable check valve (30) allows only a limited flow rate in the reverse direction, i.e. fluid flow from the stretchable fluid reservoir (23) towards the second tube (8) or the rest of the lumen (28) of the balloon holder (11). When the pressurizing fluid comes from the second tube (8), for example during deflation of the occlusion means (20), the fluid pushes the ball (35) towards the stretchable fluid reservoir (23) resulting in compression of the spring (44). Once the spring is compressed, all fluid coming from the second tube (8) pass around the ball (35) and flow into the stretchable fluid reservoir (23).

A pressure sensitive check valve (3) is located in the lumen (28) of the balloon holder (11). The pressure sensitive check valve (3) establishes fluid communication in between the lumen (11) and the pressure compensation balloon (24). The pressure sensitive check valve (3) allows all fluid flow coming from the pressure compensation balloon (24) towards the lumen (28) and hence towards the occlusion means (20) via the second tube (8) and the first tube (9).

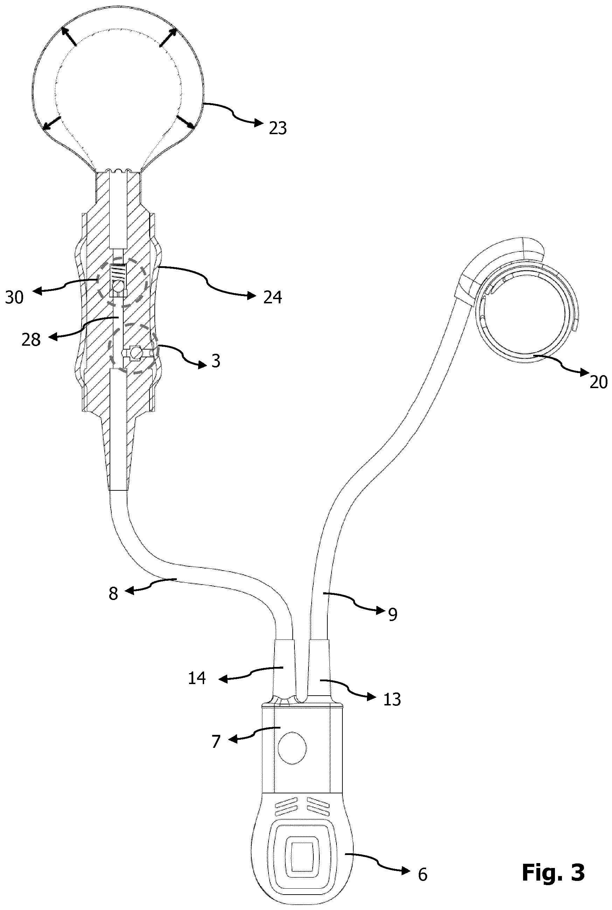

Fig. 3 shows the cross-sectional view of the artificial sphincter of Fig. 1 in the deflated state of the occlusion means (20). The occlusion means (20) is connected to first port (13) of the pump (7) via the first tube (9). The pressurizing fluid contained in the occlusion means (20) during its inflated state is transferred, via the first tube (9), partially in the bulb (6) of the pump (7) and partially in the stretchable fluid reservoir (23). The arrows shown around the stretchable fluid reservoir (23) imply that the volume of the stretchable fluid reservoir (23) has increased due to the additional volume of the pressurizing fluid transferred from the occlusion means (20), from the bulb (6) of the pump (7) and also from the pressure compensation balloon (24).

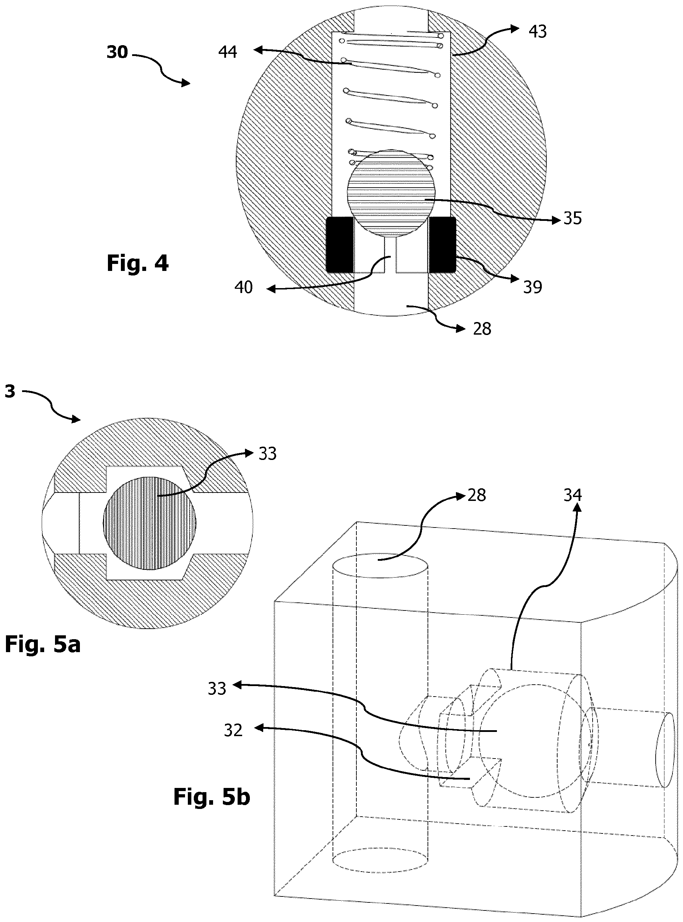

Fig. 4 shows the cross-sectional view of the semipermeable check valve (30) depicted in Fig. 2 and in Fig. 3. The semipermeable check valve (30) may comprise a spring (44) and a ball (35) which is accommodated on a recessed seating (39). The recessed seating (39) has a recess (40) for allowing restricted fluid flow from the stretchable fluid reservoir (23) towards the lumen (28) of the balloon holder (11). While the semipermeable check valve (30) allows all fluid flow coming from the lumen (28) towards the stretchable fluid reservoir (23), the recess (40) allows only very restricted flow rate in the reverse direction.

While the semipermeable check valve (30) given in Fig. 4 comprises a conventional combination of a spring (44) and a ball (35) accommodated on a seating, it may be composed of other check valve configurations. What makes the semipermeable check valve (30) unique and in particular, semipermeable, is the recess (40) which allows limited fluid flow in the reverse direction, i.e. from the stretchable fluid reservoir (23) towards the lumen (28) of the balloon holder (11).

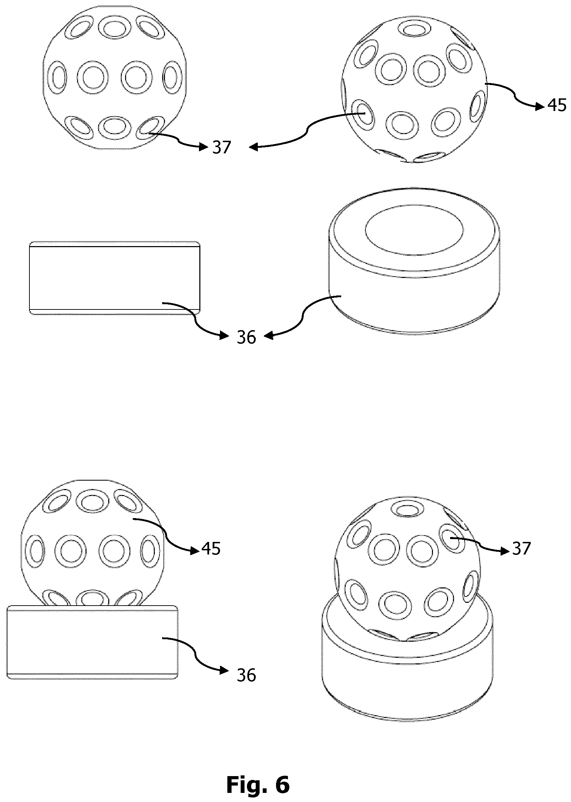

An alternative embodiment of the semipermeable check valve (30) is shown in Fig. 6 in which a cratered ball (45) is accommodated on an annular seating (36). The cratered ball (45) has several craters (37) on its outer surface such that the restricted reverse flow of the fluid from the stretchable fluid reservoir (23) can be established through the gaps in between the craters and the annular seating (36). Despite the fact that there is no need to establish a recess (40) in the annular seating (36), one or more recesses (40) may be formed on the annular seating (36) if the flow rate of the reverse flow needs to be increased.

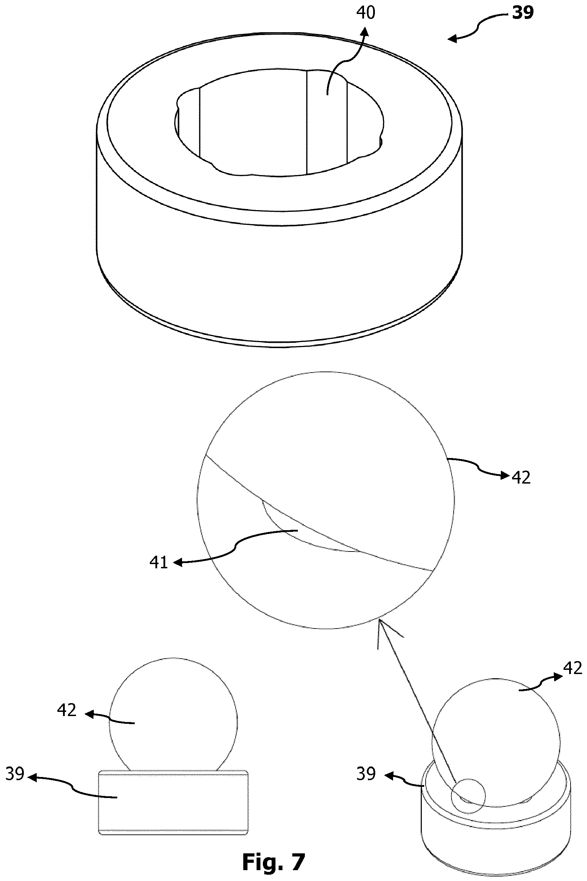

Still a further alternative embodiment of the semipermeable check valve (30) is shown in Fig. 7 in which a ball having smooth surface is accommodated on the recessed seating (39) having multiple recesses (40). In this configuration, the reverse flow, i.e. the flow from the stretchable fluid reservoir (23) towards the lumen (28) can be established through multiple gaps in between the recesses (40) of the recessed seating (39) and the ball (42).

Fig. 5a shows 2D cross sectional view of the pressure sensitive check valve given in Fig. 2 and in Fig. 3 while Fig. 5b shows 3D schematic perspective view of the same pressure sensitive check valve (3). The pressure sensitive check valve (3) is located in between the pressure compensation balloon (24) and the lumen of said balloon holder (11). The pressure sensitive check valve (3) always allows fluid flow from the pressure compensation balloon (24) towards the lumen (28) so that the fluid contained in the pressure compensation balloon (24) is freely flowable towards the inflatable occlusion means (20) and, in use, a sudden pressure increase which occurs in the abdomen of a patient is instantly conveyed to the inflatable occlusion means (20) for prevention of leaks.

The pressure sensitive check valve (3) comprises a ball (33) which floats freely in a nest (34). The density of the ball (33) is substantially close to the density of the pressurizing fluid contained in the artificial sphincter (1) proposed by the present invention. The nest (34) of the pressure sensitive check valve (3) has a pervious seat (32), as shown in Fig. 5b, at its end which is closer to the lumen (28) of the balloon holder (11). The geometry of the pervious seat (32) ensures that fluid flow towards the lumen is always guaranteed even if the ball (33) leans on the pervious seat (32) which has rectangular barriers, as shown in Fig. 5b, for ensuring passage of the fluid around the ball (33). The nest (34) has a smooth circular seat which may be blocked by the ball (33) at its other end when the fluid pressure in the lumen (28) is greater than the fluid pressure in the pressure compensation balloon (24). The check valve (3) is designated as "a pressure sensitive check valve (3)" because it blocks fluid flow towards the pressure compensation balloon (24) only when the fluid pressure in pressure compensation balloon (24) is substantially less than the fluid pressure in the lumen (28) of the balloon holder (11).

The pressure sensitive check valve (3) located in the lumen (28) of the balloon holder (11) is proximal to the second tube (8) as compared to the semipermeable check valve (30).

Reverting back to Fig. 2 in which the occlusion means (20) is depicted in the inflated state, the pressure compensation balloon (24) is in its inflated form and the stretchable fluid reservoir (23) is in its shrinked form. In this state, i.e. the occlusion means in its inflated state, the pressurizing fluid can freely flow towards the occlusion means (20) at any time. The pressure sensitive check valve (3) always allows fluid flow from the pressure compensation balloon (24) towards the lumen (28) and hence towards the occlusion means (20). Since the fluid contained in the pressure compensation balloon (24) is freely flowable towards the inflatable occlusion means (20), a sudden pressure increase which occurs in the abdomen of the patient is instantly conveyed to the inflatable occlusion means (20) resulting in better leak prevention.

Fig. 3 shows the cross-sectional view of the artificial sphincter of Fig. 1 in the deflated state of the occlusion means (20). While the pressure compensation balloon (24) in Fig. 3 is in shrinked form, inflation of the pressure compensation balloon (24) will start simultaneously with the inflation of the occlusion means (20). When the occlusion means (20) is inflated, the system reaches an equilibrium state in which the fluid pressure in the occlusion means (20), in the stretchable fluid reservoir (23) and in the pressure compensation balloon (24) are stabilized.

The equilibrium state corresponds to a state where the body canal is occluded and the patient has established his or her continence. Reverting back Fig. 5a and 5b, the ball (33) floats freely in the nest (34) once the equilibrium state is almost reached. This means that the pressure sensitive check valve (3) no longer blocks the flow of the pressurizing fluid in both directions. Once the equilibrium state is approached, the pressure compensation balloon (24) inflates and takes the form as shown in Fig. 2.

The equilibrium state is established by the semipermeable check valve (30) which allows restricted flow from the stretchable fluid reservoir (23) towards the lumen (28) of the balloon holder (11). The over pressure in the stretchable fluid reservoir (23) is smoothly and slowly reduced by the semipermeable check valve (30) which establishes the restricted reverse flow out of the stretchable fluid reservoir (23). Once the fluid pressure in the lumen (28) smoothly and slowly stabilizes, the pressure sensitive check valve (3) unblocks the flow towards the pressure compensation balloon (24) and inflates the same. The fluid pressure in the occlusion means (20), in the stretchable fluid reservoir (23) and in the pressure compensation balloon (24) stabilizes and the equilibrium state is reached with a fully inflated occlusion means (20).

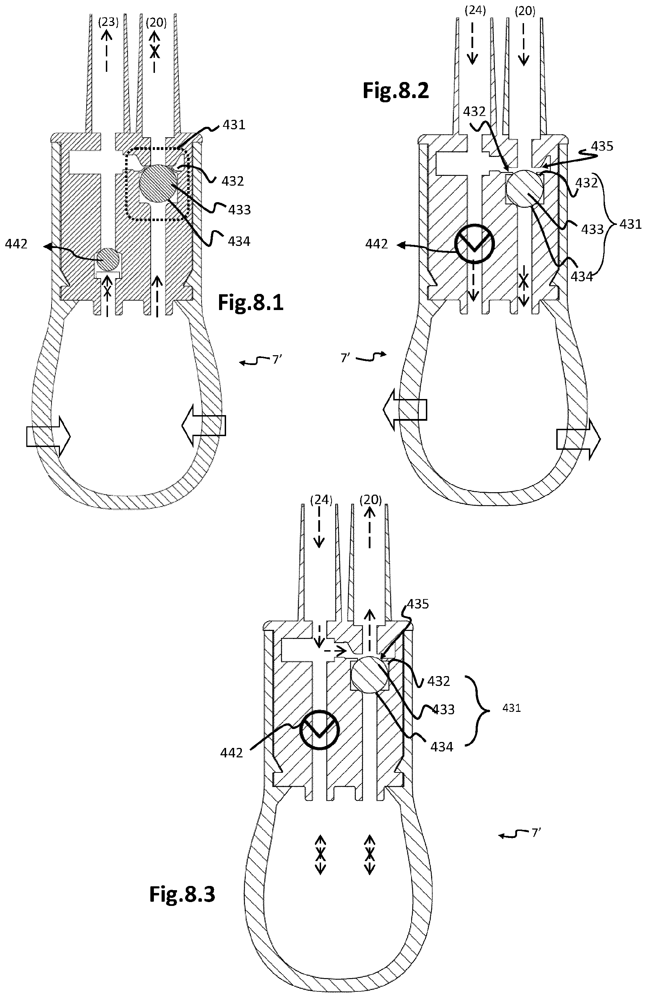

Fig. 8.1 shows a semi-schematical cross-sectional view of a pump (7) for use with the artificial sphincter according to the present invention. The pump (7) bulb is depicted at a state when the pump bulb is squeezed. For all Figs. 8.1 to 8.5, broken arrows show the flow direction of the pressurizing fluid unless the arrow is marked with an "x". In this embodiment of the pump (7), the pump has a check valve (442) which prevents flow from the pump bulb (6) towards the stretchable fluid reservoir (23). The check valve (442) only allows flow of the pressurizing fluid towards the pump (7) bulb.

Fig. 8.2 shows the pump embodiment given in Fig. 8.1 in a state when the pump bulb is released. When the patient stops squeezing the bulb, the bulb reverts back to its original shape and volume. During this state, i.e. while the bulb is reverting back to its initial shape, there is inflow of the pressurizing fluid towards the bulb as shown in Fig. 8.2. The semi-rigid check valve (431) prevents flow from the occlusion means (20) towards the pump bulb (6).

Fig. 8.2 shows the flow pathway of the pressurizing fluid in the artificial sphincter (1) during deflation of the occlusion means (20). The pressurizing fluid contained in the occlusion means (20) flows, via the first tube (9), in the bulb (6) of the pump (7). During deflation, due to the abdominal pressure, the pressurizing fluid contained in the pressure compensation balloon (24) may also flow towards the bulb (6) of the pump (7). However, each time the bulb is compressed as shown in Fig. 8.1, the fluid in the bulb flows towards the stretchable fluid reservoir (23). As the flow towards the occlusion means (20) is blocked by the semi-rigid check valve (431) as depicted in Fig. 8.1., the act of squeezing and releasing the bulb results in evacuation of the fluid contained in the occlusion means (20). In this state, the occlusion means (20) unblock the body canal and the patient may freely urinate or defecate.

Fig. 8.3 shows the pump during inflation of the occlusion means (20). During automatic inflation of the occlusion means (20), the pressurizing fluid found in the pressure compensation balloon (24) can no longer flow into the pump bulb when the bulb (6) is full. After the bulb (6) is full of the pressurizing fluid, the pressurizing fluid will gradually flow towards the occlusion means (20) and also towards the pressure compensation balloon (24) and fills both simultaneously until the occlusion means (20) is fully inflated and the patient is continent again. As the artificial sphincter (1) approaches the equilibrium state, the fluid pressure in the stretchable fluid reservoir (23) equals to the pressure in the compensation balloon (24) and in the occlusion means (20).

### Deflation of the Occlusion Means:

When the patient needs to urinate, the patient shall squeeze and release the bulb (6) of the pump a few times, typically 3 or 4 times is sufficient to transfer all fluid contained in the occlusion means (20) and in the pressure compensation balloon (24) in the stretchable fluid reservoir (23). When the bulb is squeezed as shown in Fig. 8.1, the fluid inside the bulb goes to the stretchable fluid reservoir (23). There is no fluid flow towards the occlusion means (20) as the pump has a check valve (431) preventing flow towards the occlusion means (20). However, when the bulb (6) is released, the pressurizing fluid in the occlusion means (20) is sucked out and partly enters the bulb. When the bulb is released as shown in Fig. 8.2., the bulb (6) sucks the fluid contained in the pressure compensation balloon (24). The check valve (442) allows the fluid coming from the pressure compensation balloon (24) fill in the bulb (6) of the pump (7). As each time the bulb is squeezed and released, the fluid contained in the occlusion means (20) and in the pressure compensation balloon (24), simultaneously decrease in volume and after typically 3-5 times of squeeze & release, the occlusion means (20) and the pressure compensation balloon (24) has no pressurizing fluid at all. The patient is now free to urinate.

### Inflation of the Occlusion Means:

The occlusion means is automatically inflated by the abdominal pressure of the patient as well as by and the pressure increase arising from the stretching of the walls of the stretchable fluid reservoir (23). Since the fluid previously found in the occlusion means (20) and in the pressure compensation balloon (24) were transferred to the stretchable fluid reservoir (23), the internal pressure of the stretchable fluid reservoir (23) is very high not only because the reservoir (23) is in its stretched form but also because of the abdominal pressure of the patient. Inflation of the occlusion means (20) occur gradually because of the fact that the semipermeable check valve (30) allows only a restricted reverse fluid flow towards the lumen (28) of the balloon holder (11). The semipermeable check valve (30) has tiny recesses (40) which retard the reverse flow from the stretchable fluid reservoir (23) towards the lumen (28). However, the restricted reverse fluid flow simultaneously fills the bulb (6) and the occlusion means (20). Once the bulb (6) is full, the reverse flow from the stretchable fluid reservoir (23) starts inflating the occlusion means (20) as shown in Fig. 8.3. While the bulb (6) may be squeezed and shrinked in size, the bulb (6) cannot expand more than its initial volume.

Once the restricted flow from the stretchable fluid reservoir (23) inflates the occlusion means (20), the artificial sphincter (1) according to the present invention reaches is almost-stable form and finally the reverse flow from the stretchable fluid reservoir (23) flows through the pressure compensation balloon (24) and fills in the same. The patient is now continent and the artificial sphincter (1) is now sensitive to sudden abdominal pressure changes which occur during laughing, coughing etc. The gradual and automatic inflation of the occlusion means (20) completes typically in around 3 minutes, during which the patient may freely urinate.

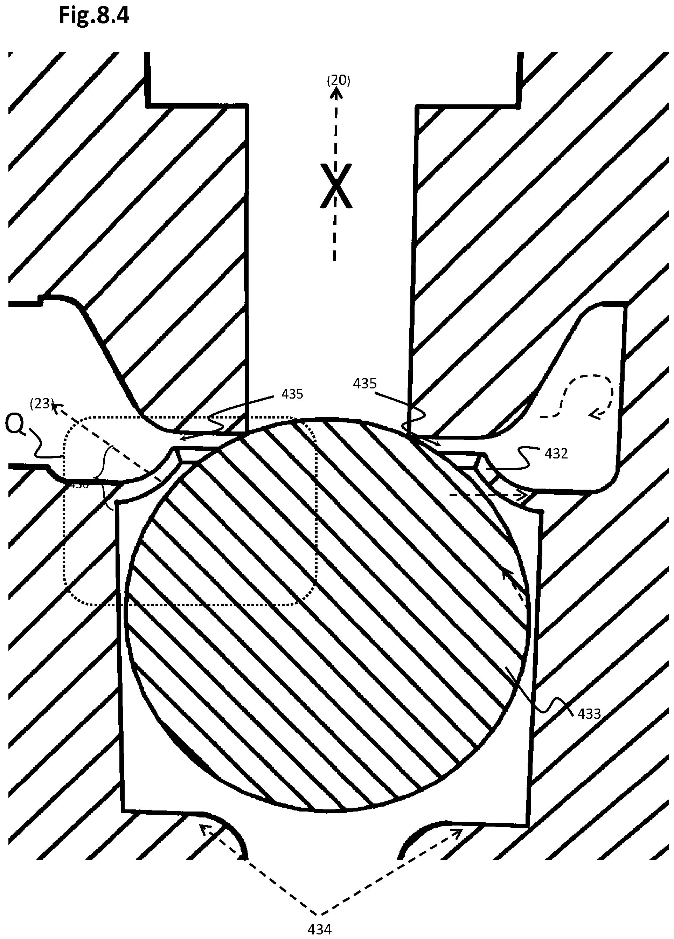

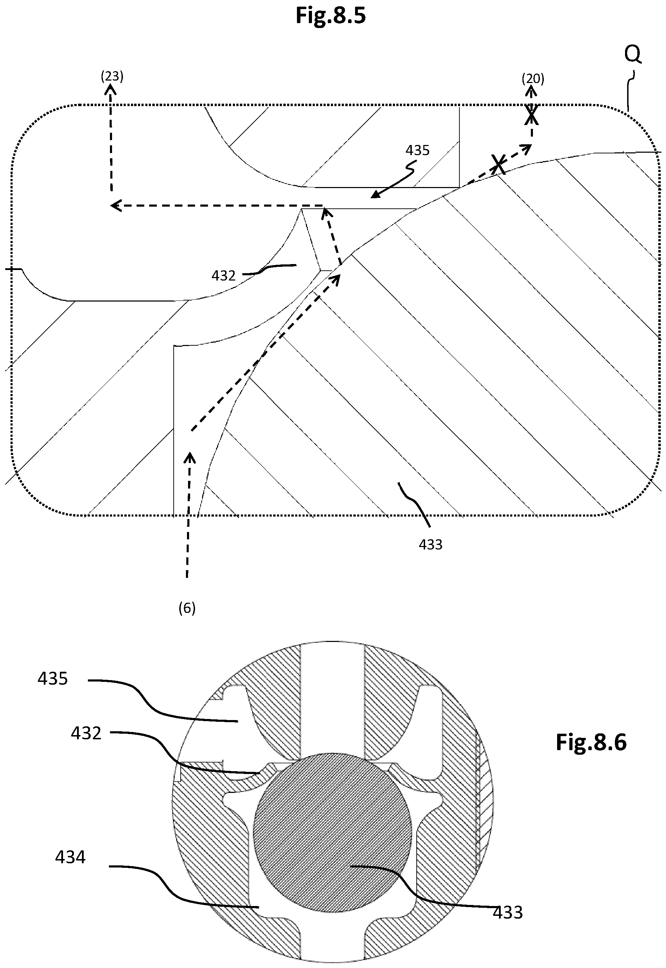

The semi-rigid check valve (431) comprises a resilient lip (432), a ball (433) and a valve seat (434) as shown in Fig. 8.1. to Fig. 8.6. The ball (433) is normally accommodated in between the valve seat (434) and resilient lips (432). In this position, semi-rigid check valve (431) the blocks flow of the pressurizing fluid towards the pump bulb (6). The ball (433) always remains on the seat (434) unless the pump bulb is squeezed. Once the bulb (6) is squeezed, the liquid pressure pushes the ball (433) and hence the resilient lips (432), resulting in dislocation of the ball and blocking the flow path towards the occlusion means (20) as illustrated in Fig. 8.4. In this position, the resilient lips (432) are bent towards the opening (435) found immediately above the resilient lips (432). While the resilient lips (432) are bent towards the opening (435), it does not block the opening (435) and allows fluid coming from around the ball (433) to pass and flow towards the stretchable fluid reservoir (23) as depicted in Fig. 8.5.

The designation of "semi-rigid check valve (431)" originates from the limited bent position of the resilient lips (432) as shown in Fig. 8.6. Once the resilient lips (432) are bent towards the opening (435), the fluid contained in the bulb passes around the ball (433) and flows towards the stretchable fluid reservoir (23) through the opening (435). Normally, all the fluid contained in the pump bulb (6) can be transferred to the stretchable fluid reservoir (23) after the patient squeeze and release the bulb a plurality of times, typically 3-4 times. As shown in the close-up view of Fig. 8.4 and Fig. 8.5 the ball (433) blocks fluid flow towards the occlusion means (20) in its dislocated position. Arrows marked with "x" mean that a flow does not exist in the direction of the arrow. The arrows given in broken lines in Fig. 8.5. shown the flow pathway of pressurizing fluid around the ball (433) when the bulb (6) is squeezed and the resilient lips (432) are bent.

## Claims

1. An artificial sphincter (1) containing, in use, a pressurizing fluid, said artificial sphincter (1) comprising:
an inflatable occlusion means (20) for occluding a body passage,
a stretchable fluid reservoir (23),
a pump means (7) having a first port (13) in fluid communication with said occlusion means (20) via a first tube (9) and a second port (14) in fluid communication with said fluid reservoir (23) via a second tube (8) for selectively transferring pressurizing fluid from said occlusion means to said reservoir to deflate said occlusion means so that a blocked body passage may be opened;
a balloon holder (11) having a lumen (28) which establishes fluid communication in between said fluid reservoir (23) and said second tube (8),
a pressure compensation balloon (24) which is in fluid communication with the lumen of said balloon holder (11) and which, in use, is to be implanted in the abdomen of a patient,
wherein the artificial sphincter (1) further comprises;
a semipermeable check valve (30) which is located in the lumen (28) of said balloon holder (11), wherein said semipermeable check valve (30) always allows fluid flow coming from the second tube (8) to pass towards said stretchable fluid reservoir (23) and wherein said semipermeable check valve (30) only allows a restricted fluid flow from the stretchable fluid reservoir (23) towards the second tube (8), **characterized in**
a pressure sensitive check valve (3) in between the pressure compensation balloon (24) and the lumen of said balloon holder (11), wherein said pressure sensitive check valve (3) always allows fluid flow from the pressure compensation balloon (24) towards the lumen (28) so that the fluid contained in the pressure compensation balloon is freely flowable towards the inflatable occlusion means (20) and, in use, a sudden pressure increase which occurs in the abdomen of a patient is instantly conveyed to the inflatable occlusion means (20) and wherein said pressure sensitive check valve (3) blocks fluid flow towards the pressure compensation balloon (24) only when the fluid pressure in pressure compensation balloon (24) is substantially less than the fluid pressure in the lumen (28) of the balloon holder (11).

2. An artificial sphincter (1) according to Claim 1 wherein the semipermeable check valve (30) has a recessed seating (39) having one or more recesses (40) so that a restricted fluid flow from the stretchable fluid reservoir (23) towards the second tube (8) is established through the recesses (40).

3. An artificial sphincter (1) according to Claim 1 wherein the semipermeable check valve (30) has an annular seating (16) and a cratered ball (45) having a plurality of craters (37) so that a restricted fluid flow from the stretchable fluid reservoir (23) towards the second tube (8) is established through the craters (37).

4. An artificial sphincter (1) according to Claim 1 wherein the pressure sensitive check valve (3) has a ball (33) located inside a nest (34), said ball allowing fluid flow from the lumen (28) towards the pressure compensation balloon (24) only during inflation of the occlusion means (20).

5. An artificial sphincter (1) according to Claim 1 wherein the pressure sensitive check valve (3) has a pervious seat (32) which is located in between the ball (33) and the lumen (28) and which ensures that the ball (33) always allows, due to its spherical geometry, passage of fluid flow from the pressure compensation balloon (24) towards the lumen (28).

6. An artificial sphincter (1) according to Claim 5 wherein a pervious seat (32) is provided in between the ball (33) and the lumen (28) such that free flow of the pressurizing fluid around the ball (33) towards the lumen is ensured.

7. An artificial sphincter (1) according to Claim 4 wherein the ball (33) has a density which is substantially equal to the density of the pressurizing fluid so that the ball freely floats freely in said nest (34).

8. An artificial sphincter (1) according to Claim 1 wherein a check valve (431) in the pump is arranged such that pressurizing fluid in the pressure compensation balloon (24) is freely flowable towards the occlusion means (20) at all times.

9. An artificial sphincter (1) according to Claim 1 wherein the reservoir (23) is made of a resilient material which may expand by way of stretching such that, in the stretched state, the pressurizing fluid contained in said reservoir (23) has a pressure more than the abdominal pressure of the patient due to stretching of the resilient material.

10. An artificial sphincter (1) according to Claim 1 wherein said pump (7) has a bulb (6) which contains pressurizing fluid and which transfers said pressurizing fluid to said reservoir (23) when compressed.

11. An artificial sphincter (1) according to Claim 1 wherein said pressure sensitive check valve (3) in the lumen (28) of the balloon holder (11) is proximal to the second tube (8) as compared to the semipermeable check valve (30).

12. An artificial sphincter (1) according to Claim 1 wherein the occlusion means (20) is sized and shaped to occlude an anal or urethral canal of a human being.

## Patentansprüche

1. Ein künstlicher Schließmuskel (1), der bei Verwendung eine Druckflüssigkeit enthält, wobei der künstliche Schließmuskel (1) folgendes umfasst:
ein aufblasbares Okklusionsmittel (20) zum Verschließen eines Körperdurchgangs,
ein dehnbares Fluidreservoir (23),
eine Pumpe (7) mit einer ersten Öffnung (13), die über einen ersten Schlauch (9) in Fluidverbindung mit dem Okklusionsmittel (20) steht, und einer zweiten Öffnung (14), die über einen zweiten Schlauch (8) in Fluidverbindung mit dem Fluidreservoir (23) steht, um selektiv Druckfluid von dem Okklusionsmittel zu dem Reservoir zu übertragen, um das Okklusionsmittel zu entleeren, so dass ein blockierter Körperdurchgang geöffnet werden kann;
einen Ballonhalter (11) mit einem Lumen (28), das eine Fluidverbindung zwischen dem Fluidreservoir (23) und dem zweiten Schlauch (8) herstellt,
einen Druckausgleichsballon (24), der in Fluidverbindung mit dem Lumen des Ballonhalters (11) steht und der bei Verwendung in den Bauchraum eines Patienten implantiert werden soll,
wobei der künstliche Schließmuskel (1) ferner umfasst:
ein halbdurchlässiges Rückschlagventil (30), das sich im Lumen (28) des Ballonhalters (11) befindet, wobei das semipermeable Rückschlagventil (30) immer einen Fluidstrom von dem zweiten Schlauch (8) zu dem dehnbaren Fluidreservoir (23) zulässt und wobei das semipermeable Rückschlagventil (30) nur einen begrenzten Fluidstrom von dem dehnbaren Fluidreservoir (23) zu dem zweiten Schlauch (8) zulässt, **dadurch gekennzeichnet, dass**
ein druckempfindliches Rückschlagventil (3) zwischen dem Druckausgleichsballon (24) und dem Lumen des Ballonhalters (11), wobei das druckempfindliche Rückschlagventil (3) immer einen Fluidfluss vom Druckausgleichsballon (24) zum Lumen (28) zulässt, so dass das im Druckausgleichsballon enthaltene Fluid frei zum aufblasbaren Okklusionsmittel (20) fließen kann und bei der Verwendung ein plötzlicher Druckanstieg, der im Abdomen eines Patienten auftritt, sofort zu dem aufblasbaren Verschlussmittel (20) geleitet wird, und wobei das druckempfindliche Rückschlagventil (3) den Fluidstrom zu dem Druckausgleichsballon (24) nur dann blockiert, wenn der Fluiddruck in dem Druckausgleichsballon (24) wesentlich geringer ist als der Fluiddruck in dem Lumen (28) des Ballonhalters (11).

2. Ein künstlicher Schließmuskel (1) gemäß Anspruch 1, wobei das semipermeable Rückschlagventil (30) einen vertieften Sitz (39) mit einer oder mehreren Vertiefungen (40) aufweist, so dass ein eingeschränkter Flüssigkeitsstrom vom dehnbaren Fluidreservoir (23) zum zweiten Schlauch (8) durch die Vertiefungen (40) hergestellt wird.

3. Künstlicher Schließmuskel (1) nach Anspruch 1, wobei das semipermeable Rückschlagventil (30) einen ringförmigen Sitz (16) und eine Kraterkugel (45) mit mehreren Kratern (37) aufweist, so dass ein begrenzter Flüssigkeitsstrom vom dehnbaren Fluidreservoir (23) zum zweiten Schlauch (8) durch die Krater (37) hergestellt wird.

4. Künstlicher Schließmuskel (1) nach Anspruch 1, wobei das druckempfindliche Rückschlagventil (3) eine Kugel (33) aufweist, die sich in einer Aufnahme (34) befindet, wobei die Kugel einen Fluidfluss aus dem Lumen (28) zum Druckausgleichsballon (24) nur während des Aufblasens des Okklusionsmittels (20) ermöglicht.

5. Künstlicher Schließmuskel (1) nach Anspruch 1, wobei das druckempfindliche Rückschlagventil (3) einen durchlässigen Sitz (32) aufweist, der sich zwischen der Kugel (33) und dem Lumen (28) befindet und der sicherstellt, dass die Kugel (33) aufgrund ihrer kugelförmigen Geometrie immer den Durchfluss von Fluidstrom vom Druckausgleichsballon (24) zum Lumen (28) ermöglicht.

6. Ein künstlicher Schließmuskel (1) gemäß Anspruch 5, wobei ein durchlässiger Sitz (32) zwischen der Kugel (33) und dem Lumen (28) vorgesehen ist, so dass ein freier Fluss des Druckfluids um die Kugel (33) herum zum Lumen gewährleistet ist.

7. Künstlicher Schließmuskel (1) nach Anspruch 4, wobei die Kugel (33) eine Dichte aufweist, die im Wesentlichen gleich der Dichte des Druckfluids ist, so dass die Kugel frei in der Aufnahme (34) schwimmt.

8. Künstlicher Schließmuskel (1) nach Anspruch 1, wobei ein Rückschlagventil (431) in der Pumpe so angeordnet ist, dass das Druckfluid im Druckausgleichsballon (24) jederzeit frei in Richtung des Okklusionsmittels (20) fließen kann.

9. Künstlicher Schließmuskel (1) nach Anspruch 1, wobei das Fluidreservoir (23) aus einem elastischen Material besteht, das sich durch Dehnen ausdehnen kann, so dass das in dem Fluidreservoir (23) enthaltene Druckfluid im gedehnten Zustand aufgrund der Dehnung des elastischen Materials einen Druck aufweist, der höher ist als der Abdominaldruck des Patienten.

10. Künstlicher Schließmuskel (1) nach Anspruch 1, wobei die Pumpe (7) einen Ballon (6) aufweist, der Druckflüssigkeit enthält und die Druckflüssigkeit bei Kompression in dem Fluidreservoir (23) überführt.

11. Künstlicher Schließmuskel (1) nach Anspruch 1, wobei das druckempfindliche Rückschlagventil (3) im Lumen (28) des Ballonhalters (11) im Vergleich zum semipermeablen Rückschlagventil (30) proximal zum zweiten Schlauch (8) angeordnet ist.

12. Künstlicher Schließmuskel (1) nach Anspruch 1, wobei das Okklusionsmittel (20) so bemessen und geformt ist, dass es einen Anal- oder Harnröhrenkanal eines Menschen verschließt.

## Revendications

1. - Sphincter artificiel (1) contenant, en utilisation, un fluide de mise sous pression, ledit sphincter artificiel (1) comprenant :
un moyen d'occlusion gonflable (20) pour occlure un passage corporel,
un réservoir de fluide extensible (23),
un moyen de pompe (7) ayant un premier orifice (13) en communication fluidique avec ledit moyen d'occlusion (20) par l'intermédiaire d'un premier tube (9) et un second orifice (14) en communication fluidique avec ledit réservoir de fluide (23) par l'intermédiaire d'un second tube (8) pour transférer de manière sélective du fluide de mise sous pression dudit moyen d'occlusion audit réservoir afin de dégonfler ledit moyen d'occlusion de telle sorte qu'un passage corporel bloqué peut être ouvert ;
un porte-ballonnet (11) ayant une lumière (28) qui établit une communication fluidique entre ledit réservoir de fluide (23) et ledit second tube (8),
un ballonnet de compensation de pression (24) qui est en communication fluidique avec la lumière dudit porte-ballonnet (11) et qui, en utilisation, doit être implanté dans l'abdomen d'un patient,
le sphincter artificiel (1) comprenant en outre :
un clapet de non-retour semi-perméable (30) qui est situé dans la lumière (28) dudit porte-ballonnet (11), ledit clapet de non-retour semi-perméable (30) permettant toujours à un écoulement de fluide provenant du second tube (8) de passer vers ledit réservoir de fluide extensible (23), et ledit clapet de non-retour semi-perméable (30) ne permettant qu'un écoulement de fluide restreint à partir du réservoir de fluide extensible (23) vers le second tube (8),
**caractérisé par** un clapet de non-retour sensible à la pression (3) entre le ballonnet de compensation de pression (24) et la lumière dudit porte-ballonnet (11), ledit clapet de non-retour sensible à la pression (3) permettant toujours un écoulement du fluide à partir du ballonnet de compensation de pression (24) vers la lumière (28) de telle sorte que le fluide contenu dans le ballonnet de compensation de pression peut s'écouler librement vers le moyen d'occlusion gonflable (20) et, en utilisation, une augmentation soudaine de pression qui se produit dans l'abdomen d'un patient est instantanément transmise au moyen d'occlusion gonflable (20), et ledit clapet de non-retour sensible à la pression (3) bloquant un écoulement de fluide vers le ballonnet de compensation de pression (24) uniquement lorsque la pression de fluide dans le ballonnet de compensation de pression (24) est sensiblement inférieure à la pression de fluide dans la lumière (28) du porte-ballonnet (11).

2. - Sphincter artificiel (1) selon la revendication 1, dans lequel le clapet de non-retour semi-perméable (30) a un siège évidé (39) ayant un ou plusieurs évidements (40) de telle sorte qu'un écoulement de fluide restreint à partir du réservoir de fluide extensible (23) vers le second tube (8) est établi à travers les évidements (40) .

3. - Sphincter artificiel (1) selon la revendication 1, dans lequel le clapet de non-retour semi-perméable (30) a un siège annulaire (16) et une bille à cratères (45) ayant une pluralité de cratères (37) de telle sorte qu'un écoulement de fluide restreint à partir du réservoir de fluide extensible (23) vers le second tube (8) est établi à travers les cratères (37).

4. - Sphincter artificiel (1) selon la revendication 1, dans lequel le clapet de non-retour sensible à la pression (3) a une bille (33) située à l'intérieur d'un nid (34), ladite bille permettant un écoulement du fluide à partir de la lumière (28) vers le ballonnet de compensation de pression (24) uniquement pendant un gonflage du moyen d'occlusion (20).

5. - Sphincter artificiel (1) selon la revendication 1, dans lequel le clapet de non-retour sensible à la pression (3) a un siège perméable (32) qui est situé entre la bille (33) et la lumière (28) et qui assure que la bille (33) permet toujours, en raison de sa géométrie sphérique, un passage d'écoulement de fluide à partir du ballonnet de compensation de pression (24) vers la lumière (28).

6. - Sphincter artificiel (1) selon la revendication 5, dans lequel un siège perméable (32) est prévu entre la bille (33) et la lumière (28) de façon à assurer un écoulement libre du fluide de mise sous pression autour de la bille (33) vers la lumière.

7. - Sphincter artificiel (1) selon la revendication 4, dans lequel la bille (33) a une densité qui est sensiblement égale à la densité du fluide de mise sous pression de telle sorte que la bille flotte librement dans ledit nid (34).

8. - Sphincter artificiel (1) selon la revendication 1, dans lequel un clapet de non-retour (431) dans la pompe est agencé de telle sorte que du fluide de mise sous pression dans le ballonnet de compensation de pression (24) peut s'écouler librement vers le moyen d'occlusion (20) à tout moment.

9. - Sphincter artificiel (1) selon la revendication 1, dans lequel le réservoir (23) est fait d'un matériau élastique qui peut s'élargir par étirement de telle sorte que, dans l'état étiré, le fluide de mise sous pression contenu dans ledit réservoir (23) a une pression supérieure à la pression abdominale du patient en raison d'un étirement du matériau élastique.

10. - Sphincter artificiel (1) selon la revendication 1, dans lequel ladite pompe (7) a une poire (6) qui contient du fluide de mise sous pression et qui transfère ledit fluide de mise sous pression jusqu'audit réservoir (23) lorsqu'elle est comprimée.

11. - Sphincter artificiel (1) selon la revendication 1, dans lequel le clapet de non-retour sensible à la pression (3) dans la lumière (28) du porte-ballonnet (11) est proximal au second tube (8) par comparaison avec le clapet de non-retour semi-perméable (30).

12. - Sphincter artificiel (1) selon la revendication 1, dans lequel le moyen d'occlusion (20) est dimensionné et formé pour occlure un canal anal ou urétral d'un être humain.
